(19) Europäisches Patentamt
European Patent Office
Office européen des brevets

(11) Publication number: 0 166 834
A1

# (12) EUROPEAN PATENT APPLICATION

(21) Application number: 84401351.6

(22) Date of filing: 26.06.84

(51) Int. Cl.⁴: C 08 G 63/62
C 08 G 63/64, C 08 L 69/00
C 07 C 39/17, C 07 C 39/42

(43) Date of publication of application:
08.01.86 Bulletin 86/2

(84) Designated Contracting States:
FR GB IT

(71) Applicant: GENERAL ELECTRIC COMPANY
1 River Road
Schenectady New York 12305(US)

(72) Inventor: Mark, Victor
701 Marigold Court
Evansville Indiana 47712(US)

(72) Inventor: Hedges, Charles Vernon
R.R. No. 3, Box 268
Mt. Vernon Indiana 47620(US)

(74) Representative: Catherine, Alain et al,
General Electric - Deutschland Munich Patent Operations
Frauenstrasse 32
D-8000 München 5(DE)

## (54) Polycarbonates exhibiting improved heat resistance.

(57) Novel polycarbonates exhibiting improved heat resistance comprised of the polymerized reaction products of (i) a carbonate precursor, and (ii) at least one cycloalkylidene containing bisphenol wherein said cycloalkylidene contains from 8 to about 16 carbon atoms in the ring structure.

EP 0 166 834 A1

Croydon Printing Company Ltd

# POLYCARBONATES EXHIBITING IMPROVED HEAT RESISTANCE

## BACKGROUND OF THE INVENTION

Polycarbonates are well known thermoplastic materials which due to their many advantageous properties find use as thermoplastic engineering materials in many commercial and industrial applications. The polycarbonates exhibit, for example, excellent properties of toughness, flexibility, impact resistance, and heat resistance. The polycarbonates are generally prepared by the coreaction of a dihydric phenol such as bisphenol-A with a carbonate precursor such as phosgene.

While the presently available conventional polycarbonates are quite useful in a wide range of applications there nevertheless exists a need, especially in applications involving high temperature environments, for polycarbonates exhibiting, to a substantial degree, substantially most of the advantageous properties of conventional polycarbonates and also exhibiting greater heat resistance than that possessed by conventional polycarbonates.

It is, therefore, an object of the instant invention to provide polycarbonates which exhibit, to a

substantial degree, substantially most of the advantageous properties of conventional polycarbonates while simultaneously exhibiting improved heat resistance.

## SUMMARY OF THE INVENTION

In accordance with the instant invention there are provided polycarbonate resins which exhibit, to a substantial degree, substantially most of the advantageous properties of conventional polycarbonate resins while simultaneously exhibiting improved heat resistance.

These polycarbonates are generally comprised of at least one repeating structural unit represented by the general formula

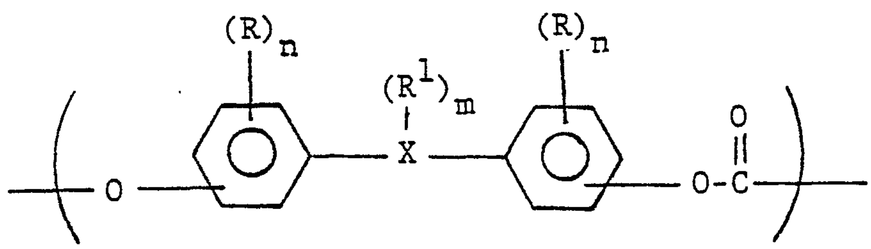

wherein:

R is independently selected from halogen radicals, monovalent hydrocarbon radicals, and monovalent hydrocarbonoxy radicals;

$R^1$ is independently selected from monovalent hydrocarbon radicals;

X represents a cycloalkylidene radical containing from 8 to about 16 ring carbon atoms;

n is independently selected from a whole number having a value of from 0 to 4 inclusive; and

m is selected from a whole number having a value of from 0 up to the number of replaceable hydrogen atoms present on X, inclusive.

## DESCRIPTION OF THE INVENTION

It has been discovered tha carbonate polymers can be obtained which exhibit improved heat resistance while simultaneously retaining, to a substantial degree, substantially most of the other advantageous properties of conventional polycarbonates.

These novel polycarbonates are derived from:

(i) a carbonate precursor; and

(ii) at least one novel dihydric phenol represented by the general formula

I. 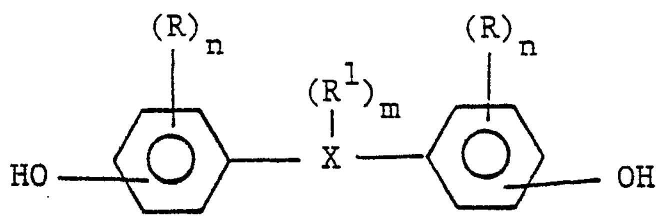

wherein:

R is independently selected from halogen radicals, monovalent hydrocarbon radicals, and monovalent hydrocarbonoxy radicals;

$R^1$ is independently selected from monovalent hydrocarbon radicals;

X represents a cycloalkylidene radical containing from 8 to about 16 ring carbon atoms;

n is independently selected from whole numbers having a value of from 0 to 4 incluisve; and

m is a whole number having a value of from 0 up to the number of hydrogen atoms present on X available for replacement.

The halogen radicals represented by R are preferably selected from chlorine and bromine.

The monovalent hydrocarbon radicals represented by R are selected from alkyl radicals, aryl radicals, aralkyl radicals, alkaryl radicals, and cycloalkyl radicals. The preferred alkyl radicals represented by R are those containing from 1 to about 8 carbon atoms. Some illustrative non-limiting examples of these alkyl radicals include methyl, ethyl, propyl, isopropyl, butyl, isobutyl, tertiary butyl, pentyl, neopentyl, and the like. The preferred aryl radicals represented by R are those containing from 6 to 12 carbon atoms, i.e., phenyl, naphthyl and biphenyl. The preferred aralkyl and alkaryl radicals represented by R are those containing from 7 to about 14 carbon atoms. Some Illustrative non-limiting examples of these alkaryl and aralkyl radicals include benzyl, tolyl, ethylphenyl, and the like. The preferred cycloalkyl radicals represented by R are those containing from 4 to about 6 ring carbon atoms and include cyclo-

butyl, cyclopentyl and cyclohexyl.

The monovalent hydrocarbonoxy radicals represented by R are preferably selected from alkoxy and aryloxy radicals. The preferred alkoxy radicals are those containing from 1 to about 8 carbon atoms and include, for example, methoxy, butoxy, propoxy, isopropoxy, and the like. The preferred aryloxy radical is phenoxy.

Preferably R is independently selected from monovalent hydrocarbon radicals, with the alkyl radicals being the preferred hydrocarbon radicals.

The monovalent hydrocarbon radicals represented by $R^1$ are selected from alkyl radicals, aryl radicals, alkaryl radicals, aralkyl radicals, and cycloalkyl radicals. The preferred alkyl radicals are those containing from 1 to about 8 carbon atoms. Some illustrative non-limiting examples of these alkyl radicals include methyl, ethyl, propyl, isopropyl, butyl, isobutyl, tertiary butyl, pentyl, neopentyl, and the like. The preferred aryl radicals are those containing from 6 to 12 carbon atoms, i.e., phenyl, naphthyl and biphenyl. The preferred aralkyl and alkaryl radicals represented by $R^1$ are those containing from 7 to about 14 carbon atoms. Some illustrative non-limiting examples of these preferred aralkyl and alkaryl radicals include benzyl, tolyl, ethylphenyl, and the like. The preferred cycloalkyl radicals rep-

resented by $R^1$ are those containing from 4 to 6 ring carbon atoms, i.e., cyclobutyl, cyclopentyl and cyclohexyl.

The preferred monovalent hydrocarbon radicals represented by $R^1$ are the alkyl radicals.

In the dihydric phenol compounds of Formula I if more than one R substituent is present on the aromatic nuclear residue they can be the same or different.

Likewise, if more than one $R^1$ substituent is present on the cycloalkylidene radical represented by X they may be the same or different.

In Formula I m preferably represents a whole number having a value of from 0 to about 6.

The preferred dihydric phenols of Formula I are the 4,4'-bisphenols.

Some illustrative non-limiting examples of the dihydric phenols represented by Formula I include:

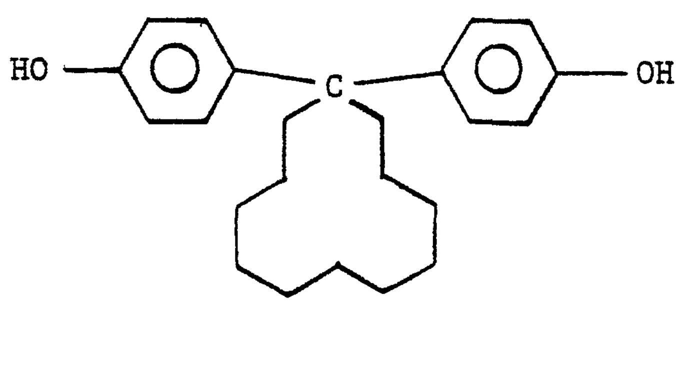

;

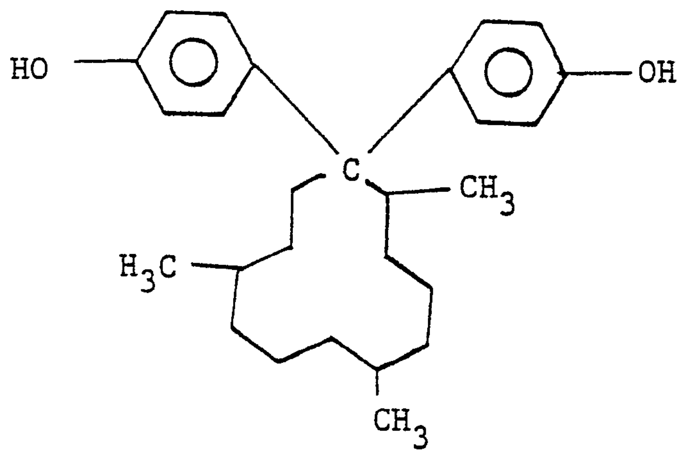

;

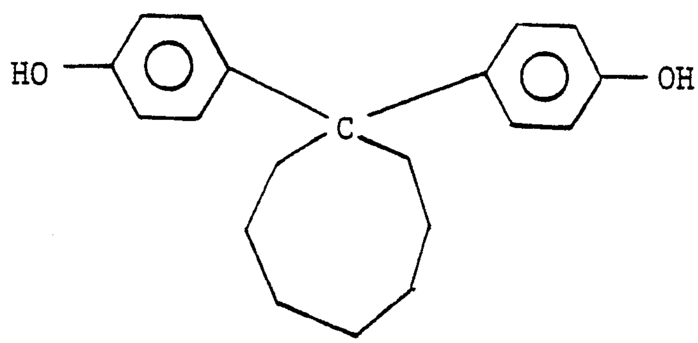

;

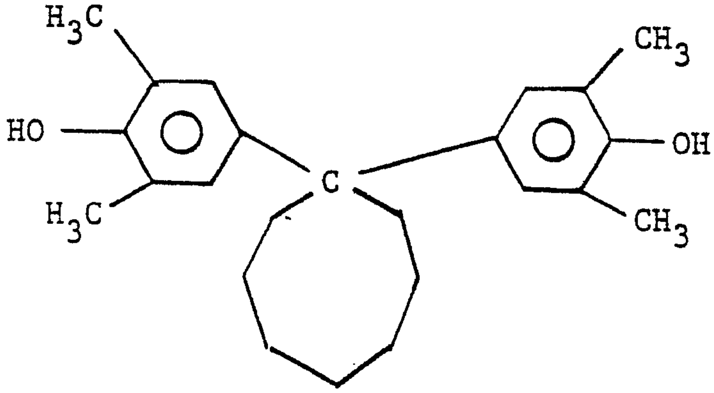

;

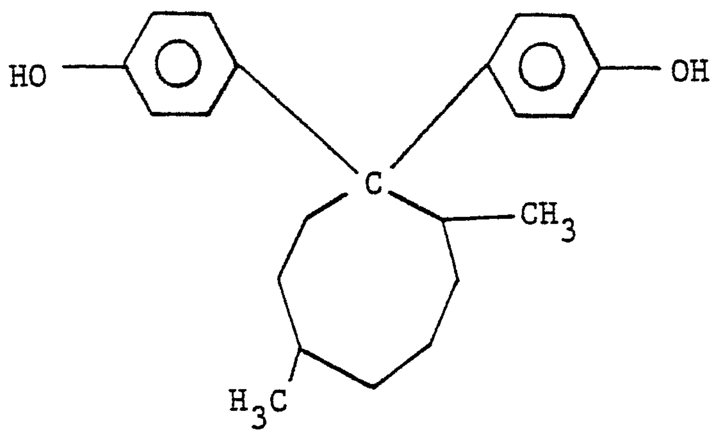

;

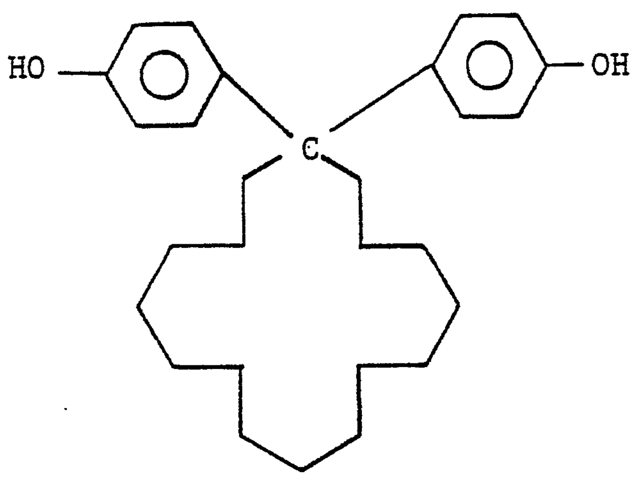

;

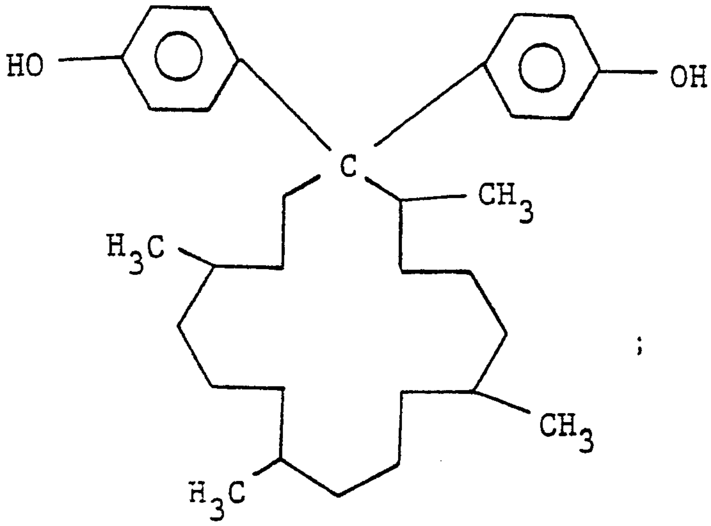

;

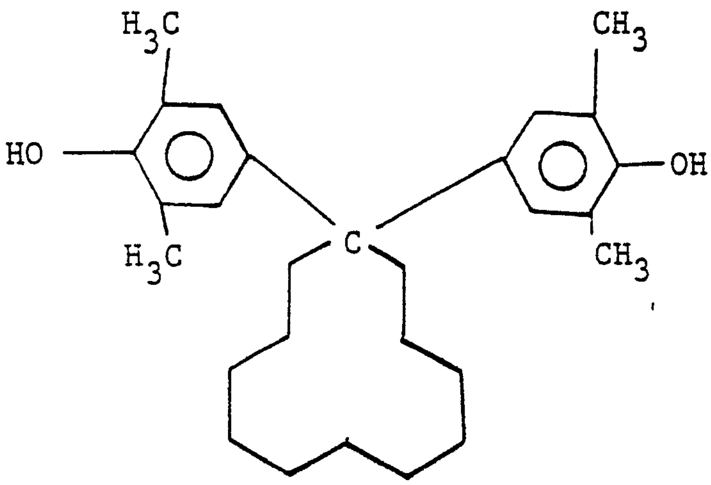

;

and the like.

The novel dihydric phenols of Formula I are prepared by the reaction of a particular ketone with a phenol in the presence of an acid catalyst, perferably in the presence of an acid catalyst and a cocatalyst such as butyl mercaptan.

The particular ketone reactant is selected from ketones represented by the general formula

II. 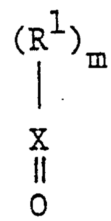

wherein $R^1$, m and X are as defined hereinafore. More particularly, the ketone of Formula II may be represented by the general formula

III. 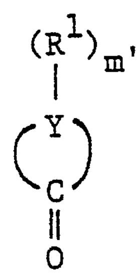

wherein $R^1$ is as defined hereinafore, Y is selected from alkylene radicals containing from 7 to about 15 carbon atoms which together with the 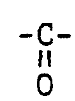 radical form a cyclic structure containing from 8 to about 16 carbon atoms, and m' represents a whole number having a value of from 0 up to the number of replaceable

hydrogen atoms present on Y.

The phenol reactants are selected from phenols represented by the general formula

IV. 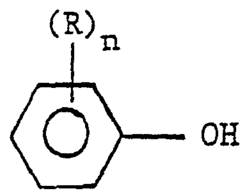

wherein R and n are as defined hereinafore.

In order to obtain the novel dihydric phenols of Formula I one mole of the ketone of Formula III is reacted with two moles of the phenol of Formula IV in the presence of an acid catalyst, and preferably in the presence of an acid catalyst and a cocatalyst such as butyl mercaptan. Generally, the phenol reactant is present in excess. Rather than utilizing only one phenol reactant a mixture of two different phenol reactants may be employed.

Some illustrative non-limiting examples of suitable acid catalysts that may be employed include hydrochloric acid, hydrobromic acid, poly(styrene sulfonic acid), sulfuric acid, benzene sulfonic acid, and the like. The phenol of Formula IV is reacted with the ketone of Formula III under conditions of temperature and pressure , and in the presence of the acid catalyst, such that coreaction between said phenol and said ketone will occur to form the dihydric phenol of Formula I. Generally, the reaction proceeds satisfactorily at about one atmosphere of pressure and

and at temperatures of from about room temperature (25°C) to about 100°C.

The amount of the acid catalysts employed is a catalytic amount. By catalytic amount is meant an amount effective to catalyze the reaction between the ketone and the phenol to produce the dihydric phenol. Generally, this amount is in the range of from about 0.1 to about 10 percent. However, in actual practice it is usually somewhat higher since the water coproduct formed in the reaction dilutes the acid catalyst and renders it somewhat less effective (slowing the reaction) than in its undiluted state.

In the preparation of the carbonate polymers of the instant invention only one dihydric phenol of Formula I may be employed, or a mixture of two or more different dihydric phenols of Formula I may be used.

The carbonate precursor that is reacted with the dihydric phenol of Formula I may be a carbonyl halide, a diaryl carbonate, or a bishaloformate. The preferred carbonate precursors are the carbonyl halides. The carbonyl halides are selected from carbonyl chloride, carbonyl bromide, and mixtures thereof. The preferred carbonyl halide is carbonyl chloride, also known as phosgene.

The novel carbonate polymers of the instant invention will contain at least one repeating structural unit represented by the general formula

V. 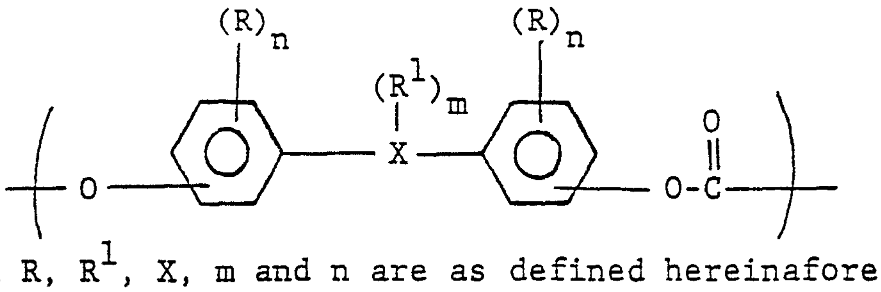

wherein R, $R^1$, X, m and n are as defined hereinafore.

These high molecular weight aromatic carbonate polymers generally have a weight average molecular weight in the range of from about 10,000 to about 200,000, preferably in the range of from about 20,000 to about 100,000.

Also included herein are the thermoplastic randomly branched polycarbonates. These randomly branched polycarbonates may be prepared by coreacting (i) a carbonate precursor, (ii) at least one dihydric phenol of Formula I, and (iii) a minor amount of a polyfunctional organic compound. The polyfunctional organic compound is generally aromatic in nature and functions as a branching agent. This polyfunctional aromatic compound contains at least three functional groups selected from hydroxyl, carboxyl, haloformyl, carboxylic anhydride, and the like. Some typical polyfunctional aromatic compounds are disclosed in U.S. Patent Nos. 3,635,895 and 4,001,184, which are hereby incorporated herein by reference. Some illustrative non-limiting examples of these polyfunctional aromatic compounds include trimellitic anhydride, trimellitic acid, trimellityl trichloride,

mellitic acid, and the like.

One method of preparing the high molecular weight aromatic carbonate polymers of the instant invention involves the heterogeneous interfacial polymerization system utilizing an aqueous caustic solution, an organic water immiscible solvent such as methylene chloride, at least one dihydric phenol represented by the general Formula I, a carbonate precursor such as phosgene, a catalyst, and a molecular weight regulator.

Another useful method for preparing the carbonate polymers of the instant invention involves the use of an organic solvent system wherein said organic solvent system may also function as an acid acceptor, at least one dihydric phenol represented by Formula I, a molecular weight regulator, a catalyst, a carbonate precursor such as phosgene, and a molecular weight regulator.

The catalysts which are employed herein can be any of the suitable catalysts that aid the polymerization reaction of a dihydric phenol with a carbonate precursor such as phosgene to produce a polycarbonate. Suitable catalysts include, but are not limited to, tertiary amines such as triethylamine, quaternary ammonium compounds, and quaternary phosphonium compounds.

The molecular weight regulators employed may be any of the known compounds which regulate the molecular weight of the carbonate polymer by a chain terminating mechanism. These compounds include, but are not limited

to, phenol, tertiary butyl phenol, chroman-I, and the like.

The temperature at which the phosgenation reaction proceeds may vary from below 0°C to above 100°C. The reaction proceeds satisfactorily at temperatures from room temperature (25°C) to about 50°C. Since the reaction is exothermic, the rate of phosgene addition or the use of a low boiling solvent such as methylene chloride may be used to control the reaction temperature.

The carbonate polymers of the instant invention may optionally have admixed therewith certain commonly known and used additives such as antioxidants; antistatic agents; fillers such as glass fibers, maica, talc, clay, and the like; impact modifiers; ultraviolet radiation absorbers such as the benzophenones, benzotriazoles, cyanoacryaltes, and the like; plasticizers; hydrolytic stabilizers such as the epoxides disclosed in U.S. Patent Nos. 3,489,716, 4,138,379 and 3,839,247, all of which are hereby incorporated herein by reference; color stabilizers such as the organophosphites disclosed in U.S. Patent Nos. 3,305,520 and 4,118,730, both of which are hereby incorporated herein by reference; flame retardants; and the like.

Some particularly useful flame retardants are the alkali and alkaline earth metal salts of sulfonic acids. These types of flame retardants are disclosed in U.S. Patent Nos. 3,933,734; 3,948,851; 3,926,908;

3,919,167; 3,909,490; 3,953,396; 3,931,100; 3,978,024; 3,953,399; 3,917,559; 3,951,910 and 3,940,366, all of which are hereby incorporated herein by reference.

Another embodiment of the instant invention is a carbonate copolymer obtained by reacting (i) a carbonate precursor, (ii) at least one dihydric phenol of Formula I, and (iii) at least one dihydric phenol represented by the general formula

VI. 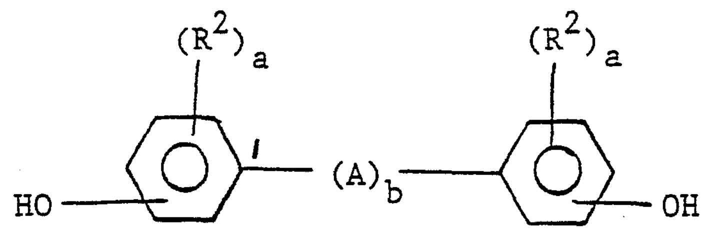

wherein:

$R^2$ is independently selected from halogen radicals, monovalent hydrocarbon radicals, and monovalent hydrocarbonoxy radicals;

a is independently selected from whole numbers having a value of from 0 to 4 inclusive;

b is either zero or one; and

A is selected from alkylene radicals, alkylidene radicals, -S-, -O-, -S-S-, $-\underset{\underset{O}{\|}}{C}-$, $-\underset{\underset{O}{\|}}{S}-$, and

$-\overset{\overset{O}{\|}}{\underset{\underset{O}{\|}}{S}}-$.

The preferred halogen radicals represenetd by $R^2$ are chlorine and bromine.

The monovalent hydrocarbon radicals represented by $R^2$ are the alkyl radicals, the aryl radicals, the aralkyl radicals, the alkaryl radicals, the aralkyl radicals, and the cycloalkyl radicals. The preferred alkyl radicals represented by R are those containing from 1 to about 8 carbon atoms. Some illustrative non-limiting examples of these alkyl radicals include methyl, ethyl, propyl, isopropyl, butyl, isobutyl, tertiary butyl, pentyl, neopentyl, and the like. The preferred aryl radicals represented by $R^2$ are those containing from 6 to 12 carbon atoms, i.e., phenyl, naphthyl, and biphenyl. The preferred aralkyl radicals and alkaryl radicals represented by $R^2$ are those containing from 7 to about 14 carbon atoms. Some illustrative non-limiting examples of these aralkyl and alkaryl radicals include benzyl, tolyl, ethylphenyl, and the like. The preferred cycloalkyl radicals represented by $R^2$ are those containing from 4 to about 6 ring carbon atoms and include cyclobutyl, cyclopentyl, cyclohexyl, methylcyclohexyl, and the like.

The monovalent hydrocarbonoxy radicals represented by $R^2$ are preferably selected from alkoxy radicals and aryloxy radicals. The preferred alkoxy radicals represented by $R^2$ are those containing from 1 to about 8 carbon atoms. Some illustrative non-limiting examples of these alkoxy radicals include methoxy, butoxy, isoprpoxy, propoxy, and the like. The preferred

aryloxy radical is phenoxy.

Preferably $R^2$ is independently selected from monovalent hydrocarbon radicals, with the alkyl radicals being the preferred monovalent hydrocarbon radicals.

The preferred alkylene radicals represented by A are those containing from 2 to about 6 carbon atoms. Some illustrative non-limiting examples of these alkylene radicals include ethylene, propylene, butylene, and the like. The preferred alkylidene radicals represented by A are those containing from 1 to about 6 carbon atoms. Some illustrative non-limiting examples of these alkylidene radicals include ethylidene, 1,1-propylidene, 2,2-propylidene, and the like.

The preferred dihydric phenols of Formula VI are those wherein b is one and A is selected from alkylene or alkylidene radicals.

In the dihydric phenol of Formula VI when more than one $R^2$ substituent is present on the aromatic nuclear residue they may be the same or different.

The more preferred dihydric phenols of Formula I are the 4,4'-bisphenols.

The dihydric phenols of Formula VI are well known in the art and are generally commercially available or may be readily prepared by known methods. These phenols are generally used in preparing conventional prior art polycarbonate resins.

Some non-limiting illustrative examples of the dihydric phenols of Formula VI include:

2,2-bis(4-hydroxyphenyl)propane (bisphenol-A);
1,1-bis(3-methyl-4-hydroxyphenyl)ethane;
2,2-bis(3,5-dimethyl-4-hydroxphenyl)propane;
2,2-bis(3,5-dibromo-4-hydroxyphenyl)propane;
bis(4-hydroxyphenyl)sulfone;
3,3-bis(3-methyl-4-hydroxyphenyl)pentane;
3,3'-diethyl-4,4'-dihydroxydiphenyl; and the like.

The amount of the dihydric phenol of Formula I utilized in this embodiment is an amount effective to improve the heat resistance, e.g., glass transition temperature, of the copolymers. Generally, this amount is in the range of from about 5 to about 90 weight percent, and preferably from about 10 to about 80 weight percent, based on the total amount of the dihydric phenols of Formulae I and VI employed.

The preferred dihydric phenol of Formula VI is 2,2-bis(4-hydroxyphenyl)propane.

The carbonate copolymers obtained by reacting (i) a carbonate precursor, (ii) at least one dihydric phenol of Formula I, and (iii) at least one dihydric phenol of Formula VI will contain at least the following repeating structural units:

V; and

VII. 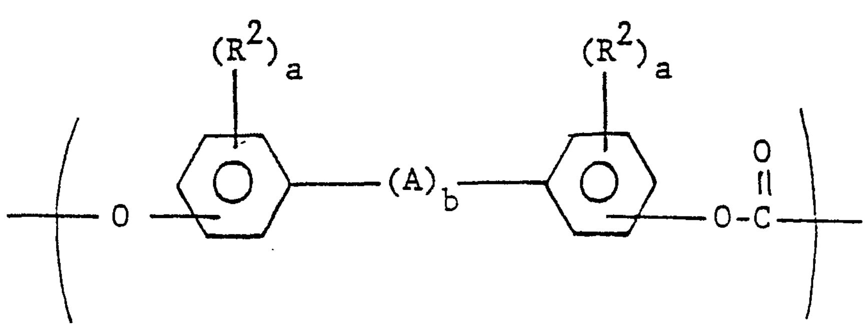

In the practice of this embodiment of the instant invention only one dihydric phenol of Formula VI may be employed or a mixture of two or more different dihydric phenols of Formula VI may be utilized.

The procedures for preparing the copolymers of this embodiment are generally similar to those used for producing the polycarbonates of the instant invention as described hereinafore. The instant carbonate copolymers may optionally have admixed therewith the various additives described hereinafore.

Yet another embodiment of the instant invention is a polycarbonate resin blend comprised of (i) at least one polycarbonate resin derived from (a) at least one dihydric phenol of Formula I, and (b) a carbonate precusror (hereinafter referred to as resin A); and (ii) at least one conventional polycarbonate resin derived from (a) at least one dihydric phenol of Formula VI, and (b) a carbonate precursor (hereinafter referred to as resin B). These blends contain an amount of resin A effective to improve the heat resistance of said blends. Generally, this amount is in the range of from about 5 to about 90 weight percent, preferably from about 10 to about 80 weight percent of resin A, based on the total amount of resins A and B present in the blends.

The blends of the instant invention may optionally contain admixed therewith the aforedescribed additives.

The instant blends may generally be prepared by

first preforming the resins A and B and thereafter physically mixing or blending these resins together. These blends may optionally contain the various additives described hereinafore.

Still another embodiment of the instant invention are copolyester-carbonates derived from (i) a carbonate precursor, (ii) at least one dihydric phenol of Formula I, and (iii) at least one difunctional carboxylic acid or a reactive derivative thereof.

Briefly stated, the copolyester-carbonates comprise recurring carbonate groups, carboxylate groups, and aromatic carbocyclic groups in the linear polymer chain, in which at least some of the carboxylate groups and at least some of the carbonate groups are bonded directly to the ring carbon atoms of the aromatic carbocyclic groups.

These copolyester-carbonates contain ester bonds and carbonate bonds in the polymer chain, wherein the amount of the ester bonds is in the range of from about 25 to about 90 mole %, preferably from about 35 to about 80 mole %. For example, 5 moles of bisphenol-A reacting completely with 4 moles of isophthaloyl dichloride and 1 mole of phosgene would give a copolyester-carbonate of 80 mole % ester bonds.

Conventional copolyester-carbonates in general, and methods for their preparation, are disclsoed in U.S. Patent 3,169,121, which is hereby incorporated by reference.

In general, any difunctional carboxylic acid conventionally used in the preparation of linear polyesters may be utilized in the preparation of the copolyester-carbonate resins of the present invention. The carboxylic acids which may be used include the aliphatic carboxylic acids, aliphatic-aromatic carboxylic acids, and aromatic carboxylic acids. These acids are disclosed in the aforementioned U.S. Patent 3,169,121.

The difunctional carboxylic acids which may be utilized in the preparation of the copolyester-carbonate resins of the instant invention generally conform to the general formula

VIII. $$R^3 - (R^4)_q - COOH$$

wherein $R^4$ is an alkylene, alkylidene, aralkylene, aralkylidene or cycloaliphatic group; an alkylene, alkylidene or cycloaliphatic group containing ethylenic unsaturation; an aromatic group such as phenylene, biphenylene, substituted phenylene, and the like; two or more aromatic groups connected through non-aromatic linkages such as alkylene or alkylidene groups; and the like. $R^3$ is either a carboxyl group or a hydroxyl group. The letter q represents one where $R^3$ is a hydroxyl group and either zero or one where $R^3$ is a carboxyl group.

Preferred difunctional carboxylic acids are the aromatic difunctional carboxylic acids, i.e., those acids of Formula VIII where q is one, $R^3$ is a carboxyl

or a hydroxyl group, and $R^4$ is an aromatic group such as phenylene, naphthylene, biphenylene, substituted phenylene, and the like. The preferred aromatic difunctional carboxylic acids are those represented by the general formula

IX. 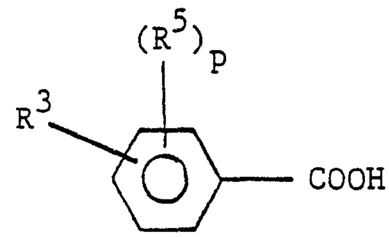

wherein $R^3$ is as defined above; $R^5$ is independently selected from halogen radicals, monovalent hydrocarbon radicals, and monovalent hydrocarbonoxy radicals; and p represents a whole number having a value of from 0 to 4 inclusive.

The preferred halogen radicals represented by $R^5$ are chlorine and bromine. The monovalent hydrocarbon radicals represented by $R^5$ are selected from alkyl radicals, aryl radicals, alkaryl radicals, aralkyl radicals, and cyclaolkyl radicals. The preferred alkyl radicals, aryl radicals, alkaryl radicals, aralkyl radicals and cyclaolkyl radicals are the same as those defined for R hereinafore. The monovalent hydrocarbonoxy radicals represented by $R^5$ are selected from alkoxy radicals and aryloxy radicals. The preferred alkoxy radicals and aryloxy radicals represented by $R^5$ are the same as those defined by R hereinafore.

Preferred radicals represented by $R^5$ are the

monovalent hydrocarbon radicals, with the alkyl radicals being the preferred monovalent hydrocarbon radiacls.

Mixtures of two or more different difunctional acids can be employed as well as single individual difunctional carboxylic acids. Therefore, where the term difunctional carboxylic acid is used herein it is meant to include mixtures of two or more different difunctional carboxylic acids as well as single difunctional carboxylic acids.

Particularly useful aromatic difunctional carboxylic acids are isophthalic acid, terephthalic acid, and mixtures thereof. A particularly useful mixture of isophthalic acid and terephthalic acid is one wherein the weight ratio of isophthalic acid to terephthalic acid is in the range of from 1:10 to 10:1.

Rather than utilizing the difunctional carboxylic acids per se it is possible, and sometimes even preferred, to employ their reactive derivatives such as, for example, the acid halides. Particularly useful acid halides are the acid chlorides. Thus, for example, instead of using isophthalic acid, terephthalic acid, or mixtures thereof it is possible to utilize isophthaloyl dichloride, terephthaloyl dichloride, or mixtures thereof.

One of the methods for preparing the copolyester-carbonates of the instant invention involves the heterogeneous interfacial polymerization system utilizing an aqueous caustic solution, an organic water immiscible solvent, at least one dihydric phenol represented by

Formula I, at least one difunctional carboxylic acid or a reactive derivative thereof, a catalyst, a molecular weight regulator, and a carbonate precursor. A preferred heterogeneous interfacial polymerization system is one which utilizes phosgene as the carbonate precursor and methylene chloride or chlorobenzene as the organic solvent.

The reaction conditions, catalysts, and chain terminators or molecular weight regulators utilized are generally the same as those described hereinafore for the preparation of the polycarbonates of the instant invention.

The copolyester-carbonate resins of the instant invention may also optionally contain admixed therewith the various additives described supra.

Another embodiment of the instant invention is a copolyester-carbonate resin derived from (i) a carbonate precursor, (ii) at least one difunctional carboxylic acid or a reactive derivative thereof, (iii) at least one dihydric phenol of Formula I, and (iv) at least one dihydric phenol of Formula VI. In this copolyester-carbonate resin the amount of the dihydric phenol of Formula I employed is an amount effective to improve the heat resistance of the resin. Generally, this amount is in the range of from about 5 to about 90 weight percent, preferably from about 10 to about 80 weight percent, based on the amount of the dihydric phenols of Formulae I and VI present.

These resins may also optionally have admixed therewith the aforedescribed additives.

Still another embodiment of the instant invention is a copolyester-carbonate resin blend comprised of (i) at least one copolyester-carbonate resin of the instant invention, i.e., one derived from (a) a carbonate precursor, (b) at least one difunctional carboxylic acid or a reactive derivative thereof, and (c) at least one dihydric phenol of Formula I (hereinafter referred to as copolyester-carbonate resin C); and (ii) at least one conventional copolyester-carbonate resin which is derived from (a) a carbonate precursor, (b) at least one difunctional carboxylic acid or a reactive derivative thereof, and (c) at least one dihydric phenol of Formula VI (hereinafter referred to as copolyester-carbonate resin D).

The blends of this embodiment contain an amount of resin C effective to improve the heat resistance of the blends. Generally this amount is in the range of from about 5 to about 90 weight percent, preferably from about 10 to about 80 weight percent, based on the amount of copolyester-carbonate resins C and D present in the blends.

These blends may also optionally contain the various aforedescribed additives.

## DESCRIPTION OF THE PREFERRED EMBODIMENT

In order to more fully and clearly illustrate the

present invention the following examples are set forth. It is intended that the examples be considered as illustrative rather than limiting the invention as disclosed and claimed herein. In the examples all parts and percents are on a weight basis unless otherwise indicated.

The following examples illustrate the preparation of the novel bisphenols of the instant invention.

EXAMPLE 1

This example illustrates the preparation of 4,4'-cyclododecylidenebisphenol.

To a 3 liter round bottom flask equipped with a stirrer, reflux condenser, thermometer and gas inlet tube, were charged 1647 grams (17.5 moles) of phenol, 478 grams (2.62 moles) of cyclododecanone, and 15 milliliters of n-butylmercaptan. Heat was applied via a heating mantel and when the reaction mixture became liquid at 58°C anhydrous hydrogen chloride was introduced untill the solution became saturated. Stirring was continued between 52 and 60° C for several hours, during which period white solids began to separate out from the reddish-orange reaction mixture. When gas chromatographic analysis of the samples taken from the slurry indicated the abscence of the macrocylic ketone, the warm reaction mixture was filtered by suction and the resultant filter cake was washed with methylenechloride in order to remove much of the excess phenol. The

filter cake was then slurried up with fresh methylene chloride, filtered and rinsed again with more solvent. Analysis by gas chromatography of the dried filter cake, which weighed 849.8 grams (2.41 moles), corresponding to a 92% yield, and melted at 207.0-208.5° C, indicated that it was 99.9% pure and that it had a retention time of 26.07 minutes relative to p-cumylphenol, which emerged at 13.91 minutes.

EXAMPLE 2

This example illustrates the preparation of 4,4'-cyclooctylidenebis(2,6-dimethylphenol).

Into a melted mixture consisting of 6.3 grams (0.05 mole) of cyclooctanone, 122.1 grams (1.0 mole) of 2,6-xylenol, and 5 milliliters of mercapotacetic acid, there was introduced gaseous hydrogen chloride until a saturated solution was obtained, while maintaining the temperature between 50 and 78°C for about 26 hours. By the end of this period there were solids formed in the melted xylenol, which solids were filtered off, washed with methylene chloride and methanol and air dried. The novel white, crystalline diphenol, which was obtained in 9.1 gram yield, melted at 253-254°C and was shown by gas chromatography (elution time was 24.35 minutes relative to 13.5 minutes for p-cumylphenol) to be 98.5% pure. The structure was confirmed by proton and 13C nmr spectroscopy.

The following examples illustrate the preparation of the polycarbonate resins of the instant invention.

EXAMPLE 3

Into a mixture of 352.2 grams (1.0 mole) of 4,4'-cyclododecylidenebisphenol, 2052 grams (9.0 mole) of bisphenol-A, 7 liters of methylene chloride, 6.5 liters of water, 28 milliliters of triethylamine, and 104.2 grams (3.5 mole%) of chroman-I (containing 10% bisphenol-A) were introduced, at ambient temperature, 1180 grams of phosgene over a period of 97 minutes while maintaining the pH of the two phase system at about 11 to 11.2 by the addition of a 25% aqueous sodium hydroxide solution. After addition of the phosgene had been terminated the methylene chloride phase was separated from the aqueous phase, washed with an excess of dilute (0.01N) aqueous HCl and then washed three times with deionized water. The polymer was precipitated with steam and dried at 95°C. The resultant polycarbonate, which had an intinsic viscosity in methylene chloride at 25°C of 0.475 dl/gm, was fed to an extruder, which extruder was operated at about 550°F, and the extrudate was comminuted into pellets.

The pellets were then injection molded at about 600°F into test bars of about 5" x 1/2" x 1/16" thick. The Heat Distortion Temperature Under Load (DTUL) of the test bars was determined according to ASTM D-648, at 264 psi. The DTUL of the test bars was 142.5°C.

EXAMPLE 4

Into a mixture of 42.1 grams (0.125 mole) of 4,4'-cyclododecylidene bisphenol, 28.5 grams (0.125 mole) of bisphenol-A, 0.7 milliliter of triethylamine, 0.12 gram (0.5 mole %) of phenol, 400 milliliters of methylene chloride, and 300 milliliters of water was introduced, at ambient temperature and a pH of about 11, i.e., 10.2-11.4, phosgene at the rate of 1 gram per minute for a period of 27 minutes while maintaining the pH of the two phase system at about 10.2 - 11.4 by the addition of a 25% aqueous sodium hydroxide solution. After addition of the phosgene had been terminated the methylene chloride phase was separated from the aqueous phase, washed with an excess of dilute (0.01N) aqueous HCl and then washed three times with deionized water. The polymer was then precipitated with methanol. The resultant polymer had a second order glass transition temperature (Tg) of above 186°C.

The following example illustrates the preparation of a conventional prior art polycarbonate falling outside the scope of the instant invention. This example is presented for comparative purposes only.

EXAMPLE 5

This example illustrates the preparation of a bisphenol-A type polycarbonate.

Into a mixture of 2283 grams of pure 4,4'-isopropylidenebisphenol (bisphenol-A) (mp 156-157°C; 10.0 mole grams), 5700 grams water, 9275 grams methylene chloride, 32.0 grams phenol and 10.0 grams of triethylamine were introduced, at ambient temperature, 1180 grams phosgene over a period of 97 minutes while maintaining the pH of the two phase system at about 11; i.e., pH 10-12.5, by simultaneously adding a 25% aqueous sodium hydroxide solution. At the end of the addition period, the pH of the aqueous phase was 11.7 and the bisphenol-A content of this phase was less than 1 part per million (ppm) as determined by ultraviolet analysis.

The methylene chloride phase was separated from the aqueous phase, washed with an excess of dilute (0.01N) aqueous HCl and then washed three times with deionized water. The polymer was precipitated by steam and dried at 95°C. The resultant, pure bisphenol-A polycarbonate, which had an intrinsic viscosity (IV) in methylene chloride at 25°C of 0.572 dl/gm., was fed to an extruder, which extruder was operated at about 550°F, and the extrudate was comminuted into pellets.

The pellets were then injection molded at about 600°F into test bars of about 5" x 1/2" x 1/16" thick. The DTUL of the test bars was determined to be 128°C. The Tg of this polycarbonate was 149°C.

The data in Examples 3-5 clearly illustrates that the polycarbonate resins of the instant invention (Examples 3-4) exhibit improved heat resistance, as measured by the second order glass transition temperature and the heat distortion temperature under load, over the conventional prior art polycarbonate resins as exemplified by Example 5.

The following example illustrates the preparation of a copolyester-carbonate resin of the instant invention.

EXAMPLE 6

To a reactor vessel there were added 400 milliliters of methylene chloride, 300 millilitersof water, 35.2 grams of 4,4'-cyclododecylidenebisphenol, 0.09 grams of phenol, and 0.28 milliliter of triethylamine. At a pH of about 11, 5.1 grams of isophthaloyldichloride were added over a 15 minute period, while maintaining the pH at about 11 by the addition of a 35% aqueous caustic solution. After the addition of the isophthaloyl dichloride was terminated 8.9 grams of phosgene were introduced over a 15 minute period while maintaining the pH at about 11 by the introduction of the 35% aqueous caustic solution. The polymer mixture was diluted with methylene chloride and the brine phase was separated. The resulting polymer containing phase was washed with HCl and then with water, and the polymer was recovered by methanol precipitation. The resultant copolyester-carbonate polymer had an intrinsic viscosity in methylene chloride at 25° C of 0.472 dl/gm. and a Tg of 240.0°C.

The following example illustrates the preparation of a conventional prior art copolyester-carbonate resin falling outside the scope of the instant invention. This example is presented for comparative purposes only.

EXAMPLE 7

To a reactor vessel were added 400 milliliters of methylene chloride, 300 milliliters of water, 34.2 grams of bisphenol-A, 0.35 gram of phenol, and 0.42 milliliter of triethylamine. At a pH of about 11, 7.6 grams of isophthaloyl dichloride dissolved in methylene chloride, 10 milliliters, were added over a 15 minute period, while maintaining the pH at about 11 by the addition of 35% aqueous caustic. After the addition of the isophthaloyl dichloride was terminated 6 grams of phosgene were introduced over a 15 minute priod, while controlling the pH at about 11 by the addition of 35% aqueous caustic solution. The polymer mixture was diluted with methylene chloride and the brine phase was separated. The resulting polymer containing phase was washed with HCl and then with water, and the polymer was then recovered by methanol precipitation. The resultant copolyester-carbonate was found to have an intrinsic viscosity of 0.530 dl/gm. and a Tg of 182.2° C.

Comparing the data of Examples 6 and 7 it is readily apparent that the copolyester-carbonate resins of the instant invention (Example 6) have a much higher Tg than do the conventional prior art copolyester-carbonate resins (Example 7).

Furthermore, the Tg of 240.0°C of the instant copolyester-carbonate resin of Example 6 is also much higher than a Tg of 199°C possessed by a conventional prior art copolyester-carbonate resin which is derived from phosgene, isophthaloyl dichloride, and cyclohexylidenebisphenol.

Obviously, other modifications and variations of the present invention are possible in light of the above teachings. It is, therefore, to be understood that changes may be made in the particular embodiment of the invention described which are within the full intended scope of the invention as defined by the appended claims.

WHAT IS CLAIMED IS:

1. Thermoplastic polymeric compositions exhibiting improved heat resistance comprised of (i) at least one thermoplastic polymer derived from:

(a) a carbonate precursor; and

(b) at least one dihydric phenol represented by the general formula

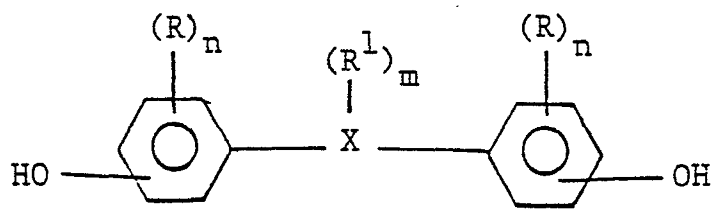

wherein:

R is independently selected from halogen radicals, monovalent hydrocarbon radicals, and monovalent hydrocarbonoxy radicals;

$R^1$ is independently selected from monovalent hydrocarbon radicals;

X represents a cycloalkylidene radical containing from 8 to about 16 ring carbon atoms;

n is independently selected from whole numbers having a value of from 0 to 4 inclusive; and

m represents a whole number having a value of from 0 up to the number of replaceable hydrogen atoms present on X.

2. The compositions of claim 1 wherein said monovalent hydrocarbon radicals represented by $R^1$ are selected from alkyl radicals, aryl radicals, alkaryl radicals, aralkyl radicals, and cycloalkyl radicals.

3. The compositions of claim 2 wherein said monovalent hydrocarbon radicals are selected from alkyl radicals.

4. The compositions of claim 3 wherein said alkyl radicals contain from 1 to about 8 carbon atoms.

5. The compositions of claim 2 wherein m is zero.

6. The compositions of claim 2 wherein said dihydric phenol is a 4,4'-bisphenol.

7. The compositions of claim 2 wherein said monovalent hydrocarbon radicals represented by R are selected from alkyl radicals, aryl radicals, aralkyl radicals, aralkyl radicals, and cycloalkyl radicals.

8. The compositions of claim 2 wherein said halogen radicals represented by R are selected from chlorine and bromine.

9. The compositions of claim 2 wherein said monovalent hydrocarbonoxy radicals represented by R are selected from alkoxy and aryloxy radicals.

10. The compositions of claim 2 wherein said carbonate precursor is phosgene.

11. The compositions of claim 10 wherein said monovalent hydrocarbon radicals represented by $R^1$ are selected from alkyl radicals.

12. The compositions of claim 11 wherein R is independently selected from alkyl radicals

13. The compositions of claim 12 wherein m is zero.

14. The compositions of claim 13 wherein n is zero.

15. The compositions of claim 14 wherein x is the cyclododecylidene radical.

16. The compositions of claim 15 wherein said dihydric phenol is 4,4'-cyclododecylidene bisphenol.

17. The compositions of claim 2 wherein said thermoplastic polymer (i) is derived from:

(a);

(b); and

(c) at least one dihydric phenol represented by the general formula

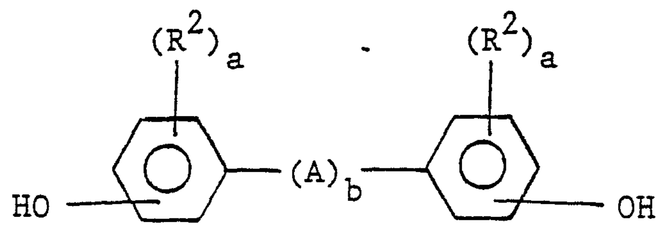

wherein:

$R^2$ is independently selected from halogen radicals, monovalent hydrocarbon radicals, and monovalent hydrocarbonoxy radicals;

A is selected from alkylene radicals, alkylidene radicals, -S-, -S-S-, -O-, $\underset{O}{\underset{\|}{-C-}}$, $\underset{O}{\underset{\|}{-S-}}$, and

$\overset{O}{\overset{\|}{\underset{\underset{O}{\|}}{-S-}}}$;

b is either zero or one; and

a is independently selected from whole numbers having a value of from 0 to 4 inclusive.

18. The compositions of claim 17 which contain an amount of dihydric phenol of (b) effective to improve the heat resistance thereof.

19. The compositions of claim 18 wherein said amount is from about 5 to about 90 weight percent, based on the amount of dihydric phenols of (b) and (c) present.

20. The compositions of claim 18 wherein said amount is from about 10 to about 80 weight percent.

21. The compositions of claim 19 wherein b is one.

22. The compositions of claim 21 wherein A is selected from alkylene radicals and alkylidene radicals.

23. The compositions of claim 22 wherein said halogen radicals represented by $R^2$ are selected from chlorine and bromine.

24. The compositions of claim 22 wherein said monovalent hydrocarbonoxy radicals are selected from alkoxy radicals and aryloxy radicals.

25. The compositions of claim 22 wherein said monovalent hydrocarbon radicals represented by $R^2$ are selected from alkyl radicals, aryl radicals, alkaryl radicals, aralkyl radicals, and cycloalkyl radicals.

26. The compositions of claim 25 wherein said monovalent hydrocarbon radicals represented by $R^2$ are selected from alkyl radicals.

27. The compositions of claim 22 wherein $R^2$ is independently selected from monovalent hydrocarbon radicals.

28. The compositions of claim 27 wherein said monovalent hydrocarbon radicals are selected from alkyl radicals, aryl radicals, aralkyl radicals, alkaryl radicals, and cycloalkyl radicals.

29. The compositions of claim 28 wherein said monovalent hydrocarbon radicals are alkyl radicals.

30. The compositions of claim 29 wherein $R^1$ is selected from alkyl radicals.

31. The compositions of claim 30 wherein R is independently selected from monovalent hydrocarbon radicals.

32. The compositions of claim 31 wherein said monovalent hydrocarbon radicals are selected from alkyl radicals, aryl radicals, aralkyl radicals, alkaryl radicals, and cyclaolkyl radicals.

33. The compositions of claim 32 wherein said monovalent hydrocarbon radicals are selected from alkyl radicals.

34. The compositions of claim 33 wherein said dihydric phenol of (b) is a 4,4'-bisphenol.

35. The compositions of claim 34 wherein m is zero.

36. The compositions of claim 35 wherein n is zero.

37. The compositions of claim 36 wherein X is a cyclododecylidene radical.

38. The compositions of claim 37 wherein said dihydric phenol of (c) is a 4,4'-bisphenol.

39. The compositions of claim 38 wherein a is zero.

40. The compositions of claim 39 wherein said dihydric phenol is bisphenol-A.

41. The compositions of claim 2 which further include (ii) at least one thermoplastic polymer derived from:

(d) a carbonate precursor; and

(e) at least one dihydric phenol represented by the general formula

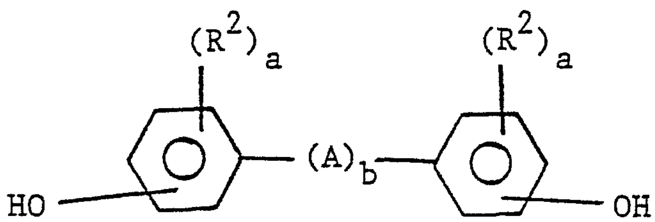

wherein:

$R^2$ is independently selected from halogen radicals, monovalent hydrocarbon radicals, and monovalent hydrocarbonoxy radicals;

A is selected from alkylene radicals, alkylidene radicals, -S-, -O-, -S-S-, $-\underset{\underset{O}{\|}}{C}-$, $-\underset{\underset{O}{\|}}{S}-$, and

$-\overset{\overset{O}{\|}}{\underset{\underset{O}{\|}}{S}}-$;

b is either zero or one; and

a is independently selected from whole numbers having a value of from 0 to 4 inclusive.

42. The compositions of claim 41 which contain an amount of polymer (i) effective to improve the heat resistance of said compositions.

43. The compositions of claim 42 where said amount is in the range of from about 5 to about 90 weight percent, based on the total amount of polymers (i) and (ii) present.

44. The compositions of claim 2 wherein said polymer of (i) is derived from:

(a);

(b); and

(f) at least one difunctional carboxylic acid or a reactive derivative thereof.

45. The compositions of claim 44 wherein said monovalent hydrocarbon radicals represented by $R^1$ are selected from alkyl radicals.

46. The compositions of claim 45 wherein said alkyl radicals contain from 1 to about 8 carbon atoms.

47. The compositions of claim 46 wherein said dihdyric phenol is a 4,4'-bisphenol.

48. The compositions of claim 47 wherein said halogen radicals represented by R are selected from chlorine and bromine.

49. The compositions of claim 47 wherein said monovalent hydrocarbonoxy radicals are selected from alkoxy and aryloxy radicals.

50. The compositionsof claim 47 wherein said monovalent hdyrocarbon radicals represented by R are

selected from alkyl radicals, aryl radicals, alkaryl radicals, aralkyl radicals, and cycloalkyl radicals.

51. The compositions of claim 50 wherein said monovalent hydrocarbon radicals are selected from alkyl radicals.

52. The compositions of claim 51 wherein m is zero.

53. the compositions of claim 52 wherein n is zero.

54. The compositions of claim 53 wherein X represents a cyclododecylidene radical.

55. The compositions of claim 54 wherein said carbonate precursor is phosgene.

56. The compositions of claim 55 wherein said difunctional carboxylic acid is selected from isophthalic acid, terephthalic acid, and mixtures thereof.

57. The compositions of claim 55 wherein said reactive derivative of said difunctional carboxylic acid is selected from isophthaloyl dichloride, terephthaloyl dichloride, and mixtures thereof.

58. The compositions of claim 44 wherein said polymer of (i) is derived from:

(a);

(b);

(f); and

(g) at least one dihydric phenol represented by the general formula

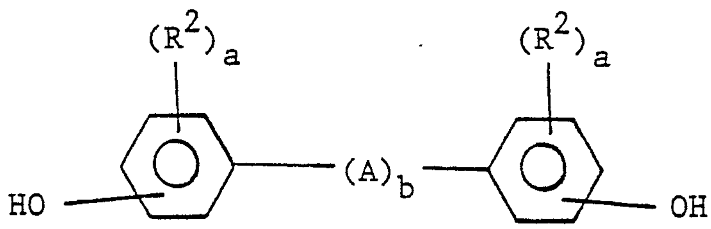

wherein:

$R^2$ is independently selected from halogen radicals, monovalent hydrocarbon radicals, and monovalent hydrocarbonoxy radicals;

A is selected from alkylene radicals, alkylidene radicals, -S-, -S-S-, -O-, $-\overset{O}{\overset{\|}{C}}-$, $-\overset{O}{\overset{\|}{S}}-$, and $-\overset{O}{\overset{\|}{\underset{\underset{O}{\|}}{S}}}-$;

b is either one or zero; and

a is independently selected from whole numbers having a value of from 0 to 4 inclusive.

59. The compositions of claim 58 which contain an amount of the dihydric phenol of (b) effective to improve the heat resistance thereof.

60. The compositions of claim 59 wherein said amount is from about 5 to about 90 weight percent, based on the amounts of dihydric phenols (b) and (g) employed.

61. The compositions of claim 60 wherein said monovalent hydrocarbon radicals represented by $R^1$ are selected from alkyl radicals.

62. The compositions of claim 60 wherein m is zero.

63. The compositions of claim 60 wherein A is selected from alkylene radicals and alkylidene radicals.

64. The compositions of claim 63 wherein b is one.

65. The compositions of claim 64 wherein said carbonate precursor is phosgene.

66. The compositions of claim 65 wherein said difunctional carboxylic acid is selected from isophthalic acid, terephthalic acid, and mixtures thereof.

67. The compositions of claim 65 wherein said reactive derivatives of said difunctional carboxylic acid are selected from isophthaloyl dichloride, terephthaloyl dichloride, and mixtures thereof.

68. The compositions of claim 44 which further contain (iii) at least one thermoplastic polymer derived from:

(h) a carbonate precursor;

(i) at least one difunctional carboxylic acid or a reactive derivative thereof; and

(j) at least one dihydric phenol represented by the general formula

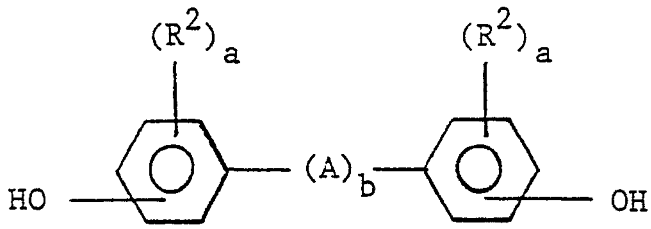

wherein:

$R^2$ is independently selected from halogen radicals, monovalent hydrocarbon radicals, monovalent hydrocarbonoxy radicals;

A is selected from alkylene radicals, alkylidene radicals, -S-, -S-S-, -O-, $-\overset{O}{\overset{\|}{S}}-$, $-\underset{\underset{O}{\|}}{\overset{O}{\overset{\|}{S}}}-$, and $-\overset{O}{\overset{\|}{C}}-$;

b is either zero or one; and

a is independently selected from whole numbers having a value of from 0 to 4 inclusive.

69. The compositions of claim 68 wherein said compositions contain an amount of polymer (i) effective to improve the heat resistance thereof.

70. The compositions of claim 69 wherein said amount is from about 5 to about 90 weight percent, based on the amount of polymers (i) and (iii) present in said compositions.

71. The compositions of claim 70 wherein A is selected from alkylene radicals and alkylidene radicals.

72. The compositions of claim 71 wherein b is one.

73. The compositions of claim 72 wherein said dihydric phenol (j) is bisphenol-A.

74. The compositions of claim 73 wherein said carbonate precursor of (a) and (h) is phosgene.

75. The compositions of claim 74 wherein said difunctional carboxylic acid of (f) and (i) is selected from isophthalic acid, terephthalic acid, and mixtures thereof.

76. The compositions of claim 74 wherein said reactive derivative of said difunctional carboxylic acid of (f) and (i) is selected from isophthaloyl dichloride, terephthaloyl dichloride, and mixtures thereof.

77. Dihydric phenols represented by the general formula

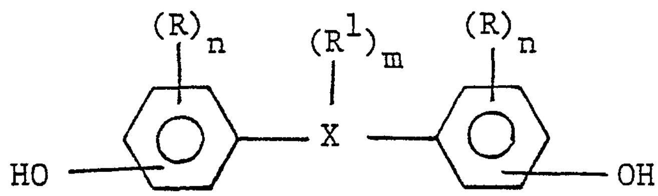

wherein:

R is independently selected from halogen radicals, monovalent hydrocarbon radicals, and monovalent hydrocarbonoxy radicals;

$R^1$ is independently selected from monovalent hydrocarbon radicals;

X represents a cycloalkylidene radical containing from 8 to about 16 ring carbon atoms;

n is independently selected from whole numbers having a value of from 0 to 4 inclusive; and

m represents a whole number having a value of from 0 up to the number of replaceable hydrogen atoms present on X.

78. The dihydric phenols of claim 77 wherein the monovalent hydrocarbon radicals represented by $R^1$ are selected from alkyl radicals, aryl radicals, aralkyl radicals, alkaryl radicals, and cycloalkyl radicals.

79. The dihydric phenols of claim 78 wherein said monovalent hydrocarbon radicals are selected from alkyl radicals.

80. The dihydric phenols of claim 79 wherein said alkyl radicals contain from 1 to about 8 carbon atoms.

81. The dihydric phenols of claim 79 wherein said halogen radicals represented by R are selected from chlorine and bromine.

82. The dihydric phenols of claim 79 wherein said monovalent hydrocarbonoxy radicals are selected from alkoxy radicals and aryloxy radicals.

83. The dihydric phenols of claim 79 wherein said monovalent hydrocarbon radicals represented by R are selected from alkyl radicals, aryl radicals, aralkyl radicals, and cycloalkyl radicals.

84. The dihydric phenols of claim 83 wherein said monovalent hydrocarbon radicals are selected from alkyl radicals.

85. The dihydric phenols of claim 79 wherein said dihydric phenols are 4,4'-bisphenols.

86. The dihydric phenols of claim 85 wherein m is zero.

87. The dihydric phenols of claim 86 wherein n is zero.

88. The dihydric phenols of claim 87 wherein X represents the cyclododecylidene radical.

89. The dihydric phenols of claim 85 wherein X represents a cyclooctylidene radical.

90. The dihydric phenols of claim 89 wherein said dihydrci phenol is 4,4'-cyclootylidenebis(2, 6-dimethylphenol).

91. The dihydric phenols of calim 88 wherein said dihydric phenol is 4,4'-cyclododecylidene bisphenol.

European Patent Office

# EUROPEAN SEARCH REPORT

0166834

Application number

EP 84 40 1351

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | DE-A-1 495 841 (FARBENFABRIKEN BAYER AG)<br>* Page 1, lines 1-9; page 3, lines 6-16; page 4, lines 1-6; claim * | 1,5-10,12-14,17-29,35,36,77,85-87 | C 08 G 63/62<br>C 08 G 63/64<br>C 08 L 69/00<br>C 07 C 39/17<br>C 07 C 39/42 |
| X | DE-A-1 570 703 (GENERAL ELECTRIC CO.)<br>* Claims 1,2; page 4, line 25 - page 5, line 10; page 10, line 5 * | 1,2,17,35,36,77,85-87,89 | |
| X | FR-B-2 119 016 (BAYER AG)<br>* Claim 1 * | 1,5,13,35 | TECHNICAL FIELDS SEARCHED (Int Cl 4)<br>C 07 C 39/00<br>C 08 G 63/00<br>C 08 L 69/00 |
| X | FR-A-2 310 370 (BAYER AG)<br>* Claim 1 * | 1,5,35,77,85-87 | |
| X | FR-A-2 175 714 (BAYER AG)<br>* Claim 1 * | 1,5,35,77,85-91 | |

--- -/-

The present search report has been drawn up for all claims

| Place of search | Date of completion of the search | Examiner |
|---|---|---|
| BERLIN | 12-02-1985 | IDEZ C.G. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

0166834

Application number

EP 84 40 1351



## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | DE-A-2 750 064 (GENERAL ELECTRIC CO.)<br>* Claim 1; page 9, lines 5-11; page 10, lines 21-25 * | 1,5,13,17,35-37,44,52-60,62-67,77,85 | |
| X | WO-A-8 000 348 (GENERAL ELECTRIC CO.)<br>* Claim 1; page 4, lines 1-8; page 5, lines 6-9 * | 1,5,44,52,54-60,77,85,86,88,89 | |
| X | WO-A-8 200 468 (GENERAL ELECTRIC AG)<br>* Claims 1-9; page 7, lines 7-14 * | 1,17,41-44,58-77,85-91 | TECHNICAL FIELDS SEARCHED (Int Cl 4) |
| X | FR-B-1 357 212 (THE DOW CHEMICAL CO.)<br>* Claims * | 77-80,83,85,86,89 | |
| X | US-A-2 883 365 (R.A. MATHES)<br>* Column 2, lines 3-31 * | 77,85-91 | |

-/-

The present search report has been drawn up for all claims

| Place of search | Date of completion of the search | Examiner |
|---|---|---|
| BERLIN | 12-02-1985 | IDEZ C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0166834

European Patent Office

Application number EP 84 40 1351

# EUROPEAN SEARCH REPORT



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 66, No. 26, 26th June 1967; Columbus, Ohio, USA; page 10823, column 1, abstract Nr. 116287 r; & JP-A-66-14751 (Sumitomo Chemical Co.), 19th August 1966<br>--- | 77,85-87,90 | |
| X | US-A-4 438 241 (V. MARK et al.)<br>* Column 1, line 49 - column 4, line 36 *<br>----- | 77-91 | |
| | | | TECHNICAL FIELDS SEARCHED (Int. Cl.4) |

The present search report has been drawn up for all claims

| Place of search | Date of completion of the search | Examiner |
|---|---|---|
| BERLIN | 12-02-1985 | IDEZ C.G. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document